(19) Europäisches Patentamt
European Patent Office
Office européen des brevets

(11) Veröffentlichungsnummer: **0 218 863 B1**

(12) **EUROPÄISCHE PATENTSCHRIFT**

(45) Veröffentlichungstag der Patentschrift: **15.04.92**

(51) Int. Cl.5: **C12N 9/04**, C12P 7/42, //(C12N9/04,C12R1:225)

(21) Anmeldenummer: **86111812.3**

(22) Anmeldetag: **26.08.86**

# (54) Mikrobiologisch hergestellte D(-)-Mandelat-Dehydrogenase, Verfahren zu ihrer Gewinnung und ihre Verwendung.

(30) Priorität: **15.10.85 DE 3536662**

(43) Veröffentlichungstag der Anmeldung:
**22.04.87 Patentblatt 87/17**

(45) Bekanntmachung des Hinweises auf die Patenterteilung:
**15.04.92 Patentblatt 92/16**

(84) Benannte Vertragsstaaten:
**BE CH DE FR GB LI NL SE**

(56) Entgegenhaltungen:
**EP-A- 0 000 560**
**EP-A- 0 024 547**
**EP-A- 0 130 288**

**APPLIED MICROBIOLOGY BIOTECHNOLOGY, Band 21, 1985, Seiten 7-15, Springer Verlag; W. HUMMEL et al.: "D-2-hydroxyisocaproate dehydrogenase from Lactobacillus casei"**

(73) Patentinhaber: **Degussa Aktiengesellschaft**
**Weissfrauenstrasse 9**
**W-6000 Frankturt am Main 1(DE)**

Patentinhaber: **Gesellschaft für Biotechnologische Forschung mbH (GBF)**
**Mascheroder Weg 1**
**W-3300 Braunschweig-Stöckheim(DE)**

(72) Erfinder: **Hummel, Werner, Dr.**
**Claudiusstrasse 11**
**W-5177 Titz(DE)**
Erfinder: **Schütte, Horst**
**Nordring 29 a**
**W-3332 Salzgitter 51(DE)**
Erfinder: **Kula, Maria-Regina, Prof. Dr.**
**Selgenbusch 12**
**W-5162 Niederzier/Hambach(DE)**
Erfinder: **Leuchtenberger, Wolfgang, Dr.**
**Geschwister-Scholl-Strasse 1**
**W-6454 Bruchköbel(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

CHEMICAL ABSTRACTS, Band 99, Nr. 23, 5. Dezember 1983, Seiten 609-610, Zusammenfassung Nr. 193133w, Columbus, Ohio, US; W. HUMMEL et al.: "Large scale production of D-lactate dehydrogenase for the stereospecific reduction of pyruvate and phenylpyruvate", & EUR. J. APPL. MICROBIOL. BIOTECHNOL. 1983, 18(2), 75-85

CHEMICAL ABSTRACTS, Band 99, Nr. 21, 21. November 1983, Seite 340, Zusammenfassung Nr. 172451h, Columbus, Ohio, US; C. HILLS et al.: "Mutant strains of Acinetobacter calcoaceticus possessing additional mandelate dehydrogenases. Identification and preliminary characterization of the enzymes", & BIOCHEM. J. 1983, 209(2), 379-86

## Beschreibung

Ein Gegenstand der Erfindung ist ein bisher nicht beschriebenes Enzym, das die folgende Umsetzung katalysiert:

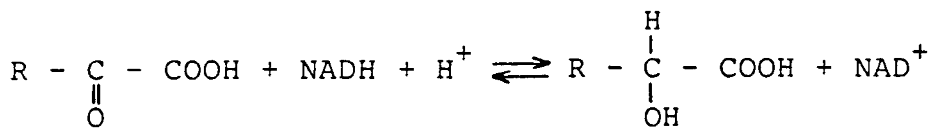

D-Konfiguration

Neben R = 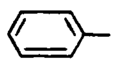

werden auch verschiedene gerad- oder verzweigtkettige aliphatische und araliphatische Reste R angenommen.

Besonders gut angenommen wird das Substrat Benzoylformiat (Phenylglyoxylat), das mit guter Aktivität stereospezifisch zu D(-)-Mandelat reduziert wird. Der Wasserstoff für die Reduktionsreaktion wird von dem Coenzym NADH (Nicotinamidadenin-dinucleotid) geliefert. Das Gleichgewicht der Umsetzung liegt auf der Seite der D(-)-Mandelsäure, so daß - insbesondere bei kontinuierlicher Reaktionsführung unter ständiger Regenierung des Coenzyms - in hoher Ausbeute Benzoylformiat in D(-)-Mandelsäure umgewandelt werden kann.

Die erfindungsgemäße mikrobiologisch hergestellte D(-)-Mandelat-Dehydrogenaseist durch die folgenden physikalischchemischen Eigenschaften gekennzeichnet:

l) Reaktivität:
sie reagiert in Gegenwart von NADH (Nicotinamid-adenindinucleotid) mit Benzoylformiat unter Bildung von D(-)-Mandelat und in Gegenwart von $NAD^+$ mit D(-)-Mandelat unter Bildung von Benzoylformiat;
2) Substratspezifität:
sie reduziert besonders gut Benzoylformiat, daneben auch andere aliphatische und araliphatische 2-Ketocarbonsäuren, und oxidiert besonders gut D(-)-Mandelat, daneben auch andere aliphatische und araliphatische D-2-Hydroxycarbonsäuren;
3) Optimaler pH-Wert:
der optimale pH-Wert für die Reduktionsreaktion ist 6,0 ± 0,5, der optimale pH-Wert für die Oxidationsreaktion ist 8,5;
4) pH-Stabilität:
zeigt nach einwöchiger Lagerung bei 4 ° C und einem pH zwischen 5 und 7,5 eine Restaktivität von 85 %;
5) Optimale Temperatur:
die optimale Temperatur beträgt 55 ° C bei einem pH-Wert von 6,0;
6) Temperaturbeständigkeit:
l5 Minuten bei einem pH-Wert von 6,0 bei 50 ° C behandelt beträgt die Restaktivität 90 %;
7) Aktivität:
zeigt eine spezifische Aktivität von ca. 2l00 U/mg Protein;
8) Einfluß von Inhibitoren:
wird durch $HgCl_2$, $CuSO_4$ oder Mercuri-p-chlorbenzoat stark inhibiert:
9) Molekulargwicht:
das Molekulargewicht beträgt 60 000 ± 5 000 (ermittelt durch Gelfiltration);
l0) Molekulargewicht der Untereinheit:
das Molekulargewicht der Untereinheit beträgt 30 000 ± 3 000 (bestimmt durch SDS-Elektrophorese);
ll) $K_M$-Wert:
der $K_M$-Wert für die Reduktionsreaktion für das Substrat Benzoylformiat bei pH 7,0 beträgt 0,22 mM, der $K_M$-Wert für die Oxidationsreaktion für das Substrat D(-)-Mandelat bei pH 8,0 0,5 mM.

Die erfindungsgemäße D(-)-Mandelat-Dehydrogenase kann aus Lactobacillus curvatus DSM 20 0l9 gewonnen werden.

Ein weiterer Gegenstand der Erfindung ist daher ein Verfahren zur Gewinnung der D(-)-Mandelat-Dehydrogenase, welches dadurch gekennzeichnet ist, daß man Lactobacillus curvatus DSM 20 0l9 in einem

wäßrigen Nährmedium, das eine Quelle für Kohlenstoff und Stickstoff, Thiamin und Mineralsalze enthält, bei einem pH zwischen 5,5 und 6,5 und einer Temperatur zwischen 30 und 37 °C anaerob kultiviert, die Zellmasse abtrennt und das Enzym aus den Zellen isoliert.

Ein letzter Gegenstand der Erfindung ist schließlich die Verwendung der erfindungsgemäßen D(-)-Mandelat-Dehydrogenase zur Herstellung von D(-)-Mandelsäure aus Benzoylformiat.

Zur Gewinnung des erfindungsgemäßen Enzyms wurde zunächst ein Screening mit 45 Stämmen der Familie Lactobacillaceae (Lactobacillus, Leucostonoc und Pediococcus) vorgenommen. Die Stämme wurden für dieses Screening im 300 ml-Maßstab unter Bedingungen angezogen, wie sie für den jeweiligen Stamm im DSM-Katalog empfohlen werden, üblicherweise im DSM-Medium Nr. II (MRS-Medium) bei 30 oder 37 °C.

Nach 20-stündigem Wachstum wurde die Zellmasse durch Zentrifugation (20 Minuten bei l0 000 UpM) geerntet, in Kaliumphosphatpuffer (0,l M; pH 7,5) suspendiert (4 ml Pufferlösung pro l g Bakterienfeuchtmasse) und dann in üblicher Weise in einem Laborschüttler aufgeschlossen. Die unlöslichen Zellbestandteile und die Glasperlen wurden durch Zentrifugation (2 Minuten bei l2 000 UpM) abgetrennt, und der Überstand (Rohextrakt) wurde auf enzymatische Aktivität getestet. Zur Erkennung des Enzyms wurde ein photometrischer Test benutzt. Der jeweilige Testansatz enthielt
l ml Kaliumphosphatpuffer (0,l M; pH 7,0),
20 µl NADH (0,2 mM Endkonzentration),
20 µl Benzoylformiat (2 mM Endkonzentration) und limitierende Mengen an Rohextrakt (l bis 20 µg Protein).

Es wurde die Abnahme der Extinktion von NADH bei 340 nm gemessen. Von den erhaltenen Werten wurde ein Nullwert abgezogen, der erhalten wurde, wenn der Test in Abwesenheit von Benzoylformiat vorgenommen wurde. Die Enzymaktivität wird in internationalen Einheiten angegeben, wobei eine Einheit (U) die Abnahme von l µMol NADH pro l Minute bedeutet.

Es zeigte sich, daß sieben der geprüften Mikroorganismen eine deutliche NADH-abhängige Aktivität bei der Reduktion von Benzoylformiat zu D(-)-Mandelat aufwiesen. Die Enzymaktivitäten der Rohextrakte sind in der Tabelle I zusammengestellt.

Tabelle 1: Screening auf D(-)-Mandelat-Dehydrogenase

| Stamm | DSM-Nr. | U/mg | U/l | Stereospezifität an C-2 |
|---|---|---|---|---|
| Lactobacillus curvatus | 20 019 | 0,17 | 75 | D |
| L. delbrueckii | 20 074 | 0,08 | 43 | D |
| L. bulgaricus | 20 080 | 0,17 | 68 | D |
| L. casei | 20 178 | 0,13 | 55 | D |
| L. brevis | 20 054 | 0,08 | 58 | D |
| L. fructivorans | 20 203 | 0,12 | 65 | D |
| L. parvus | 20 177 | 0,11 | 55 | D |

Zur Bestimmung der Stereospezifität der Enzyme wurde mit den Rohextrakten die Oxidation von D(-)- bzw. L(+)-Mandelat in Gegenwart von $NAD^+$ mit folgendem Testansatz geprüft:
1 ml Kaliumphosphatpuffer (0,1 M; pH 8,0),
20 µl $NAD^+$ (2,5 mM im Test),
20 µl D(-)- bzw. L(+)-Mandelsäure (9,5 mM im Test) und limitierende Mengen an Rohextrakt (5 bis 50 µg

Protein) im Test.

Der Stamm Lactobacillus curvatus DSM 20 019 zeigte im Screening die höchste Aktivität und wurde daher für die Gewinnung des erfindungsgemäßen Enzyms ausgewählt.

Zur Gewinnung der erfindungsgemäßen D(-)-Mandelat-Dehydrogenase wird Lactobacillus curvatus DSM 20 019 in folgendem Medium angezogen:

| | |
|---|---|
| Glucose | 20 g |
| Hefeextrakt | 5 g |
| Universalpepton | 10 g |
| Fleischextrakt | 5 g |
| Diammoniumhydrogencitrat | 2 g |
| Natriumacetat | 5 g |
| Magnesiumsulfat | 0,1 g |
| Mangansulfat | 0,05 g |
| Dikaliumhydrogenphosphat | 2 g |
| destilliertes Wasser | 1 l. |

Der pH-Wert dieser Lösung wird auf 6,5 eingestellt, dann wird für 15 Minuten bei 121 °C (2 bar) sterilisiert. Der Mikroorganismus wird anaerob kultiviert; dazu reicht es aus, wenn das Medium mit Stickstoff überlagert wird. Im 10 l-Maßstab wird das Medium nach Erreichen der Bruttemperatur von 30 °C mit 300 ml einer 24 Stunden alten Vorkultur beimpft. Die Aktivität der D(-)-Mandelat-Dehydrogenase erreicht nur während eines kurzen Zeitraumes maximale Werte, bei längerem Wachstum fällt die Aktivität dann wieder ab.

Im 5000 l-Maßstab kann ein l0 l-Ansatz als Vorfermenter verwendet werden. Aus diesem Fermenter erhält man dann ca. 25 kg Bakterienfeuchtmasse. Der pH-Wert, der im Verlauf des Wachstums absinkt, wird in den Fermentern mit konzentriertem Ammoniak bei 5,5 gehalten. Die Biomasse kann bei -20 °C ohne größere Verluste an Aktivität für einige Monate zwischengelagert werden.

Die erfindungsgemäße D(-)-Mandelat-Dehydrogenase läßt sich durch einen Aufschluß der Zellen nach herkömmlichen Methoden, z.B. Ultraschallbehandlung oder Naßvermahlung, und Abtrennen der unlöslichen Zellfragmente in Form eines Rohextraktes gewinnen. Die Zellbruchstücke werden beispielsweise durch einen ersten Flüssig-Flüssig-Verteilungsschritt in einem wäßrigen Zwei-Phasen-System aus l0 % (w/w) Polyethylenglykol (MG 6 000), 8 % (w/w) Phosphatpuffer für pH 8,0 und 5 000 ml Rohextrakt in einem l0 kg-Ansatz abgetrennt. Die Oberphase enthält dann die Hauptmenge der D(-)-Mandelat-Dehydrogenase.

Die Oberphase wird dann einem zweiten Flüssig-Flüssig-Verteilungsschritt unterworfen. Dazu wird die enzymhaltige Oberphase (3 890 ml) mit 8 % (w/v) Phosphatpuffer für pH 6,l und 0,3 M Natriumchlorid, berechnet auf ein Endvolumen von 7 780 ml, versetzt und l Stunde gerührt. Die D(-)-Mandelat-Dehydrogenase findet sich nun in der Unterphase des sich ausbildenden Polyethylenglykol/Salzlösungs-Systems.

Zur weiteren Reinigung kann nun diese salzhaltige Unterphase diafiltriert und einer DEAE-Ionenaustausch-Chromatographie unterworfen werden.

Die Erfindung wird durch die nachfolgenden Beispiele näher erläutert:

Beispiel l: Züchtung des Mikroorganismus

Lactobacillus curvatus DSM 20 0l9 wurde in l0 Litern des oben bereits genannten Mediums angezogen. Der pH-Wert dieses Mediums wurde auf 6,5 eingestellt, dann wurde es für Minuten bei l2l °C (2 bar) sterilisiert. Das Medium wurde mit Stickstoff überlagert, auf die Bruttemperatur von 30 °C erwärmt und mit 300 ml einer 24 Stunden alten Vorkultur beimpft. Der Verlauf der Enzymaktivität in Abhängigkeit von der Wachstumszeit wurde geprüft, indem zu verschiedenen Zeiten Proben entnommen wurden, und nach Aufschluß der Zellen die Aktivität der im Rohextrakt enthaltenden D(-)-Mandelat-Dehydrogenase bestimmt wurde. Die Aktivität des Enzyms erreichte nach etwa l5 Stunden Wachstum ein Maximum und fiel bei längerem Wachstum wieder deutlich ab. Nach 20 Stunden Wachstum wurden durch Zentrifugation (20 Minuten bei l0 000 UpM) 50 g Bakterienfeuchtmasse erhalten.

Beispiel 2: Isolierung und Reinigung des Enzyms

2 000 g Bakterienfeuchtmasse aus einer 5 000 l-Kultivierung 2ö wurden in l00 mM Phosphatpuffer für pH 7,5 unter Zusatz von 0,l Volumenprozent 2-Mercaptoethanol zu einer 40 gewichtsprozentigen Zellsuspension mit einem Endvolumen von 5 000 ml suspendiert. Der pH-Wert der Suspension wurde überprüft und mit verdünnter Kalilauge auf 7,5 eingestellt. Die Zellinhaltsstoffe wurden aus der auf 4 °C gekühlten Suspension mit Hilfe einer Glasperlenmühle (Hersteller: Fa. Bachofen; Dyno-Mill Typ KDL) freigesetzt. Dazu wurde der 600 ml fassende Mahlbehälter mit 0,25 bis 0,5 mm großen Glasperlen so gefüllt, daß sich ein Schüttvolumen von 5l0 ml ergab. Der Zellaufschluß wurde bei einer Rührwellendrehzahl von 3 000 UpM und einer Durchflußrate von 5 l/h durchgeführt. Der Kühlmantel und das Rührwellenlager wurden während des Laufes mit einer Ethylenglykollösung von -20 °C gekühlt, um eine Erwärmung der Suspension weitgehend zu vermeiden. Nach 3 Durchläufen war ein Desintegrationsgrad von über 90 % erreicht. Der pH-Wert der Suspension wurde mit verdünnter Kalilauge auf 7,0 eingestellt.

Es wurde ein wäßriges Zwei-Phasen-System hergestellt, welches l0 % (w/w) Polyethylenglykol (MG 6 000), 8 % (w/w) Phosphatpuffer für pH 8,0 und 5 000 ml der homogenisierten Suspension in einem Gesamtsystem von l0 kg enthielt. Zur Einstellung des Verteilungsgleichgewichts wurde das Zwei-Phasen-System eine Stunde lang gerührt, dann wurde es durch Zentrifugation getrennt. Die Oberphase (3 890 ml) enthielt über 90 % der insgesamt vorliegenden D(-)-Mandelat-Dehydrogenase. Die Unterphase enthielt die Zellbruchstücke und die unter diesen Bedingungen extrahierten Fremdproteine und wurde verworfen.

Die Oberphase wurde nun mit 8 % (w/v) Phosphatpuffer für pH 6,l und 0,3 M Natriumchlorid, berechnet auf ein Endvolumen von 7 780 ml, versetzt und eine Stunde lang gerührt. Das sich ausbildende Polyethylenglykol/Salzlösung-System ließ sich in einem Absetzgefäß in etwa l Stunde vollständig trennen. Die salzhaltige Unterphase (3 620 ml) enthielt etwa 86 % der insgesamt vorliegenden D(-)-Mandelat-Dehydrogenase.

Die abgetrennte Unterphase wurde mit einem Hohlfaser-System (Hersteller: Romicon; PM l0; l,8 $ft^2$) konzentriert und durch Zugabe von 5 mM Kaliumphoshatpuffer für pH 6,5 auf eine Endkonzentration von 5 mM diafiltriert.

Die konzentrierte und diafiltrierte Enzymlösung wurde auf eine mit DEAE-Sephacell gepackte Säule (5 cm × l4 cm) aufgepumpt. Der Ionenaustauscher war zuvor gegen einen Puffer äquilibriert worden, der 5 mM Kaliumphosphatpuffer für pH 6,5 und 0,l Volumenprozent 2-Mercaptoethanol enthielt. Die Säule wurde anschließend mit Startpuffer nachgewaschen, dann wurde das Enzym mit einem linearen Gradienten (2 × 800 ml) von 0 bis 0,5 M Natriumchlorid in Startpuffer eluiert. Die D(-)-Mandelat-Dehydrogenase eluierte mit ca. 0,l M Natriumchlorid. Die aktiven Fraktionen wurden durch Ultrafiltration aufkonzentriert, mit 50 Gewichtsprozent Glycerin versetzt und bei -20 °C aufbewahrt. Das Ergebnis der Aufreinigungsschritte ist in der Tabelle 2 zusammengefaßt. Das aufgereinigte Enzym hat eine spezifische Aktivität von etwa 2 l00 U/mg Protein.

Tabelle 2: Reinigung der D(-)-Mandelat-Dehydrogenase

| Reinigungsschritt | Volumen | Protein | Totalaktivität | spez. Aktivität | Ausbeute | Anreicherung |
|---|---|---|---|---|---|---|
| | ml | mg | U | U/mg | % | -fach |
| Rohextrakt | 5 000 | 115 000 | 340 000 | 2,96 | 100 | 1 |
| Oberphase 1 | 3 890 | 4 079 | 323 000 | 79,2 | 95 | 26,8 |
| Unterphase II | 3 620 | 3 475 | 312 400 | 89,9 | 91,9 | 30,4 |
| Diafiltration | 540 | 2 821 | 275 000 | 97,5 | 80,9 | 32,9 |
| DEAE-Cellulose | 455 | 112 | 237 700 | 2122,0 | 70,0 | 717,0 |

Test mit Benzoylformiat

Reinigung aus 2 000 g Lactobacillus curvatus-Feuchtmasse

Enzymtest:

| | |
|---|---|
| 2 850 µl | 0,1 M Phosphatpuffer für pH 6,0 |
| 50 µl | 14 mM NADH-Lösung |
| 100 µl | 30 mM Benzoylformiat-Lösung |
| 3 000 µl | |

Start der Reaktion mit Enzym

Beispiel 3: Abhängigkeit der Reaktionsgeschwindigkeit der enzymatisch katalysierten Umsetzung vom pH-Wert:

Die Reaktionsgeschwindigkeit der Reduktion von Benzolyformiat zu D(-)-Mandelat in Gegenwart der D(-)-Mandelat-Dehydrogenase wurde in Abhängigkeit vom pH-Wert der Reaktionslösung untersucht. Der Testansatz (3,00 ml) hatte folgende Zusammensetzung: 0,25 mM NADH; l,5 mM Benzoylformiat, limitierende Mengen an Enzym, 0,l M Puffer verschiedener Zusammensetzung und verschiedener pH-Werte. Das Enzym hat ein pH-Optimum zwischen pH 5,5 und pH 6,5. Der pH-Wert wurde im Reaktionsgemisch gemessen.

Die Reaktionsgeschwindigkeit der Dehydrogenierung von D(-)-Mandelat zu Benzoylformiat, katalysiert durch die D(-)-Mandelat-Dehydrogenase wurde gleichfalls in Abhängigkeit vom pH-Wert untersucht. Der Testansatz (3,00 ml) hatte folgende Zusammensetzung: 4,5 mM $NAD^+$, 2 mM D(-)-Mandelat, limitierende Mengen an Enzym, 0,l M Puffer unterschiedlicher Zusammensetzung. Die Dehydrogenierungsreaktion zeigt ein Optimum bei pH 8,5.

Beispiel 4: Lagerstabilität der D(-)-Mandelat-Dehydrogenase in Abhängigkeit vom pH-Wert.

D(-)-Mandelat-Dehydrogenase wurde in 0,l M Puffer unterschiedlicher Zusammensetzung bei einer Proteinkonzentration von 5 mg/ml für eine Woche bei 4 °C inkubiert. Danach wurde die Restaktivität, wie in Beispiel 3 beschrieben, unter Verwendung von 0,l M Phosphatpuffer für pH 6 bestimmt. Dabei zeigte sich eine gute pH-Stabilität im Bereich von pH 5 bis 7,5. Nach einer Woche waren noch 85 % der Aktivität nachweisbar, in Phosphatpuffer bei pH 6,5 sogar 98 %.

Beispiel 5: Temperaturstabilität der D(-)-Mandelat-Dehydrogenase

Gereinigte D(-)-Mandelat-Dehydrogenase (DEAE-Sephacell-Peak 2l22 U/mg) wurde in Gegenwart von 0,l M Phosphatpuffer für pH 6,0, der 5 mg/ml Rinderserumalbumin enthielt, bei verschiedenen Temperaturen inkubiert und zu bestimmten Zeiten wurde die Restaktivität ermittelt. Bei einer Temperatur von 50 °C betrug die Restaktivität nach l5 Minuten noch etwa 90 %. Bei höheren Temperaturen wurde das Enzym schnell inaktiviert.

Beispiel 6: Einfluß der Temperatur auf die Enzymaktivität

Die Reaktionsgeschwindigkeit der Reduktion von Benzoylformiat zu D(-)-Mandelat wurde in Abhängigkeit von der Reaktionstemperatur gemessen. Die maximale Reaktionsgeschwindigkeit wurde bei 55 °C erreicht. Bei der Standardmeßtemperatur von 30 °C betrug die Reaktionsgeschwindigkeit etwa 85 % des Maximalwertes. Über 55 °C ging die Reaktionsgeschwindigkeit aufgrund gleichzeitiger Denaturierung des Enzyms stark zurück.

Beispiel 7: Bestimmung des Molekulargewichts der D(-)-Mandelat-Dehydrogenase und Ermittlung der Untereinheiten

Das Molekulargewicht des nativen Enzyms wurde durch Gelfiltration an Superose l2 ermittelt. Die an ein FPLC-System gekoppelte Säule (l,0 cm × 30 cm) wurde mit einer Durchflußrate von 0,4 ml/Minute betrieben, wobei als Probegut l00 µl des an DEAE-Sephacell gereinigten Enzyms diente. Als Eichproteine wurden Cytochrom c, Pepsin, Ovalbumin, Rinderserumalbumin (BSA); D-2-Hydroxyisocaproat-Dehydrogenase, L-2-Hydroxyisocaproat-Dehydrogenase, Aldolase, L-Alanin-Dehydrogenase und L-Leucin-Dehydrogenase aus Bacillus cereus und Ferritin verwendet. Das Molekulargewicht der D(-)-Mandelat-Dehydrogenase beträgt 60 000 ± 5 000 Dalton. Durch Gelelektrophorese in Gegenwart von Natriumdodecylsulfat (SDS) konnte die Größe und Anzahl der Untereinheiten des Enzyms bestimmt werden. Das Molekulargewicht der Untereinheit beträgt 30 000 ± 3 000 Dalton. Die D(-)-Mandelat-Dehydrogenase besteht demnach aus zwei identischen Untereinheiten. Für die Eichkurve wurde Hämoglobin, β-Lactoglobulin, Chymotrypsinogen, Pepsin, Ovalbumin und BSA verwendet.

Beispiel 8: Einflüsse von verschiedenen Reagenzien und Metallionen auf die enzymatische Aktivität der D(-)-Mandelat-Dehydrogenase

Die Reaktionsgeschwindigkeit der Reduktion von Benzoylformiat zu D(-)-Mandelat wurde in Gegenwart von verschiedenen Reagenzien und Metallionen gemessen. Das Enzym wurde zu diesem Zweck zunächst 5 Minuten bei 20 °C mit dem jeweiligen Inhibitor bzw. Metallsalz inkubiert und dann die verbliebene Volumenaktivität unter Normalbedingungen ermittelt. Die D(-)-Mandelat-Dehydrogenase wird durch Anwesenheit von 0,l mM $HgCl_2$, $CuSO_4$ oder Mercuri-p-chlorbenzoat stark inhibiert, während die Anwesenheit

anderer Reagenzien selbst bei l mM keinen großen Einfluß hat. Das vollständige Ergebnis zeigt die Tabelle 3.

Tabelle 3

| Inhibition der D(-)-Mandelat-Dehydrogenase (- = nicht getestet) | | | |
|---|---|---|---|
| Reagenz | Relative Aktivität (%) | | |
| | 0,1 mM | 1 mM | 10 mM |
| ohne Zusatz | 100 | 100 | 100 |
| $MgCl_2$ | 99 | 89 | 81 |
| $CrCl_2$ | 95 | 90 | - |
| $CuSO_4$ | 6,4 | 6,1 | - |
| $CoSO_4$ | 97 | 91 | - |
| $CdCl_2$ | 93 | 82 | - |
| $K_2Cr_2O_7$ | 99 | - | - |
| $FeCl_3$ | 91 | - | - |
| $ZnCl_2$ | 94 | 88 | - |
| $NiCl_2$ | 95 | 91 | 68 |
| $Na_2MoO_4$ | 90 | 88 | 73 |
| $HgCl_2$ | 0 | 0 | 0 |
| EDTA | 94 | 93 | 87 |
| CITRAT | | | |
| 1,10 Phenanthrolin | 96 | 96 | - |
| 2,2-Dipyridil | 100 | 99 | 86 |
| Jodacetamid | 98 | 96 | 91 |
| KCN | 84 | 81 | 71 |
| Mercuri-p-chlorbenzoat | 6 | - | - |
| 2-Mercaptoethanol | 96 | 93 | 90 |
| Dithiothreitol | 100 | 100 | 100 |

Beispiel 9: Abhängigkeit der Reaktionsgeschwindigkeit von den Substratkonzentrationen

Die Abhängigkeit der Reaktionsgeschwindigkeit für die Reduktion von Benzoylformiat zu D(-)-Mandelat von der Konzentration des Coenzyms NADH wurde in folgendem Testansatz untersucht:
0,l M Phosphatpuffer für pH 7,0; 6 mMol Benzoylformiat, limitierende Mengen an Enzym (angereichertes Präparat, nach DEAE-Cellulose-Chromatographie, siehe Tabelle 2); die NADH-Konzentration im Testansatz wurde im Bereich von 0,0l bis 0,30 mM variiert. Es zeigte sich, daß die optimale Reaktionsgeschwindigkeit bei 0,25 mM erreicht wird. Der $K_M$-Wert beträgt 0,036 mM.

Die Reduktion von Benzoylformiat zu D(-)-Mandelat in Abhängigkeit von der Benzoylformiat-Konzentration wurde in folgendem Testansatz untersucht:
0,l M Phosphatpuffer für pH 7,0; 0,25 mM NADH und limitierende Mengen an Enzym. Die Benzoylformiat-Konzentration wurde im Bereich von 0,02 mM bis 8 mM variiert. Es zeigte sich, daß die optimale Reaktionsgeschwindigkeit bei l,5 mM erreicht wird. Der $K_M$-Wert beträgt 0,22 mM.

Die Reduktion verschiedener 2-Ketocarbonsäuren wurde in Abhängigkeit von der Ketosäurekonzentration untersucht. Dazu wurde folgender Testansatz verwendet:
0,l M Phosphatpuffer für pH 7,0, 0,23 mM NADH mit limitierenden Mengen an Enzym (angereichertes Präparat, nach DEAE-Cellulose Chromatographie, siehe Tabelle 2). Die 2-Ketosäurekonzentration wurde jeweils im Bereich von 0,05 bis 9 mM variiert und die Abnahme der Extinktion durch das bei der Reaktion verbrauchte NADH bei 340 nm gemessen. Die Anfangsreaktionsgeschwindigkeit wurde nach der Michaelis-Menten-Gleichung ausgewertet. Die gefundenen kinetischen Konstanten $V_{max}$ und $K_M$ sind in der Tabelle 4 zusammengefaßt.

Tabelle 4: Substratspezifität der D(-)-Mandelat-Dehydrogenase

| Substrat | max. Anfangs-Reaktionsgeschwindigkeit $V_{max}$ (%) (relativ zu Benzoylformiat) | $K_M$-Wert (M) |
|---|---|---|
| 2-Ketobyturat | 51 | $5,5 \times 10^{-4}$ |
| 2-Ketovalerat | 76 | $1,7 \times 10^{-4}$ |
| 2-Ketocaproat | 74 | $1,0 \times 10^{-4}$ |
| 2-Ketooctanoat | 6 | $3,5 \times 10^{-4}$ |
| 2-Keto-3-methylbutyrat | 176 | $1,8 \times 10^{-4}$ |
| 2-Keto-3-methylvalerat | 119 | $9,5 \times 10^{-5}$ |
| 2-Ketoisocaproat | 76 | $9,0 \times 10^{-5}$ |
| 2-Keto-4-methylmercapto-butyrat | 68 | $1,1 \times 10^{-4}$ |
| Trimethylpyruvat | 15 | $5,4 \times 10^{-3}$ |
| Benzoylformiat | 100 | $2,2 \times 10^{-4}$ |
| Phenylpyruvat | 63 | $1,5 \times 10^{-4}$ |
| 4-Hydroxyphenylpyruvat | 4 | $6,5 \times 10^{-4}$ |
| 3-(3',4') Dihydroxyphenylpyruvat | 4 | $3,7 \times 10^{-4}$ |

Die Abhängigkeit der Reaktionsgeschwindigkeit für die Dehydrogenierung des D(-)-Mandelats von der $NAD^+$-Konzentration wurde in folgendem Testansatz untersucht:

0,l M Tris/HCl-Puffer für pH 8,5, 2 mM D(-)-Mandelat, limitierende Mengen an Enzym. Die $NAD^+$-Konzentration wurde im Bereich von 0,05 mM bis 6 mM variiert und die Zunahme der Extinktion durch das bei der Reaktion entstehende NADH bei 340 nm gemessen. Es zeigte sich, daß der optimale Umsatz bei

einer Konzentration von 3 mM erreicht wird. Der $K_M$-Wert für $NAD^+$ beträgt 0,20 mM.

Die Abhängigkeit der Reaktionsgeschwindigkeit der Dehydrogenierung von D(-)-Mandelat von der D(-)-Mandelat-Konzentration wurde in folgendem Testansatz untersucht:

0,l M Phosphatpuffer für pH 8,0, 3 mM $NAD^+$ und limitierende Mengen an Enzym. Die Konzentration des D(-)-Mandelats wurde im Bereich von 0,l bis 20 mM variiert. Die Extinktion des bei der Reaktion entstehenden NADH wurde bei 340 nm gemessen. Es zeigte sich, daß der optimale Umsatz bei einer Konzentration von 6 mM erreicht wird. Der $K_M$-Wert für D(-)-Mandelat beträgt 0,5 mM.

Die Abhängigkeit der Reaktionsgeschwindigkeit der Dehydrogenierung von D-2-Hydroxycarbonsäuren von der Konzentration verschiedener D-2-Hydroxycarbonsäuren wurde in folgendem Testansatz untersucht:

0,l M Phosphatpuffer für pH 8,0, 3 mM $NAD^+$ und limitierende Mengen an Enzym. Die Konzentration der 2-Hydroxysäuren wurde im Bereich von 0,25 bis 300 mM variiert. Sofern keine chirale D-2-Hydroxysäure zur Verfügung stand, wurde das Racemat eingesetzt. Die Extinktion des bei der Reaktion entstehenden NADH wurde bei 340 nm gemessen. Die Anfangsreaktionsgeschwindigkeit wurde nach Michaelis-Menten ausgewertet und die kinetischen Konstanten $V_{max}$ und $K_M$ ermittelt. Die gefundenen kinetischen Konstanten sind in der Tabelle 5 zusammengefaßt.

Tabelle 5: Substratspezifität der D(-)-Mandelat-Dehydrogenase

| Substrat | max. Anfangs-Reaktionsgeschwindigkeit $V_{max}$ (%) (relativ zu D(-)-Mandelat) | $K_M$-Wert[a)] (M) |
|---|---|---|
| D,L-2-Hydroxyvalerat | 128 | $1{,}3 \times 10^{-2}$ |
| D,L-2-Hydroxycaproat | 67 | 1,8 c $10^{-3}$ |
| D,L-2-Hydroxyoctanoat | 3 | $2{,}2 \times 10^{-3}$ |
| D,L-2-Hydroxyisocaproat | 132 | $1{,}1 \times 10^{-3}$ |
| D-2-Hydroxy-4-methylmercapto-butyrat | 118 | $3{,}8 \times 10^{-3}$ |
| D,L-2-Hydroxy-4-methylmercapto-butyrat | 142 | $1{,}1 \times 10^{-3}$ |
| D,L-Phenyllactat | 22 | $2{,}5 \times 10^{-3}$ |
| D-Mandelat | 100 | $5 \times 10^{-4}$ |
| D,L-Mandelat | 100 | $4 \times 10^{-4}$ |
| L-Mandelat | 0 | - |

a) Für die Konzentration des D-Enantiomers in den D,L-Verbindungen wurden 50 % angenommen.

Beispiel I0: Kontinuierliche Herstellung von D(-)-Mandelsäure

Eine Synthese von chiralen Hydroxysäuren im kontinuierlichen Betrieb ist möglich in einem Enzymmembranreaktor unter Verwendung von molekulargewichtsvergrößertem, an Polyethylenglykol (PEG) gebundenem NADH. Das PEG-NADH wird gemäß DE-PS 2 84l 4l4 hergestellt. Das modifizierte Coenzym und die eingesetzten Enzyme Formiat-Dehydrogenase (zur Coenzym-Regenerierung) und D(-)-Mandelat-Dehydrogenase werden durch eine Ultrafiltrationsmembran YM 5 im Reaktor (CECl, Firma Amicon) zurückgehalten, während die niedermolekularen Bestandteile der Reaktionslösung (nichtumgesetztes Substrat, Produkt, Puffer) laufend aus der Lösung entfernt werden (Verweilzeit 2 Stunden). Das Reaktorvolumen wird konstant gehalten, indem in gleichem Maße aus einem Resevoir 50 mM Benzoylformiat in Puffer (0,l M Na-Formiat, pH 7,0) nachdosiert wird, wie das Ultrafiltrat den Reaktor verläßt.

Das Reaktorvolumen betrug l0 ml, es enthielt im einzelnen:

300 mM Natriumformiatlösung (pH 7,0)
l00 mM Tris-HCl (pH 7,0)
0,2 mM $PEG_{20\ 000}$-NADH
2 U/ml Formiat-Dehydrogenase (Präparat gemäß Kroner et al. (l982) J. Chem. Technol. Biotechnol.32, l30-l37)
2 U/ml Mandelat-Dehydrogenase (Präparat nach DEAE-Cellulose-Chromatographie; siehe Tabelle 2)
50 mM Benzoylformiat

Der Umsetzungsgrad wurde bestimmt, indem der Drehwert α der Produktlösung polarimetrisch gemessen wurde (Polarimeter 24l, Firma Perkin-Elmer; Messung bei 436 nm (Hg) bei 27 °C) Die Produktkonzentration kann dann aus einer Eichkurve ermittelt werden, die mit käuflichem D(-)-Mandelat (Firma Sigma M 2500) erstellt wurde.

Tabelle 6 zeigt, daß man Umsätze bis praktisch l00 % erreichen kann.

Tabelle 6

| Kontinuierliche Umsetzung von Benzoylformiat zu D (-)-Mandelat | | | |
|---|---|---|---|
| Reaktionszeit [Stunden] | Drehwert [Grad] | Produkt-Konzentration [mM] | Umsatz [%] |
| 2 | -0,98 | 25 | 51 |
| 5 | -1,85 | 48 | 96 |
| 10 | -1,92 | 50 | 100 |
| 20 | -1,92 | 50 | 100 |
| 30 | -1,92 | 50 | 100 |
| 40 | -1,92 | 50 | 100 |
| 50 | -1,92 | 50 | 100 |
| 60 | -1,92 | 50 | 100 |
| 70 | -1,92 | 50 | 100 |
| 80 | -1,92 | 50 | 100 |

## Patentansprüche

1. Mikrobiologisch hergestellte D(-)-Mandelat-Dehydrogenase, gekennzeichnet durch die folgenden physikalisch-chemischen Eigenschaften:
   l) Reaktivität:
   sie reagiert in Gegenwart von NADH (Nicotinamid-adenin-dinucleotid) mit Benzoylformiat unter Bildung von D(-)-Mandelat und in Gegenwart von $NAD^+$ mit D(-)-Mandelat unter Bildung von Benzoylformiat;
   2) Substratspezifität:
   sie reduziert besonders gut Benzoylformiat, daneben auch andere aliphatische und araliphatische 2-Ketocarbonsäuren, und oxidiert besonders gut D(-)-Mandelat, daneben auch andere aliphatische und araliphatische D-2-Hydroxycarbonsäuren;
   3) Optimaler pH-Wert:
   der optimale pH-Wert für die Reduktionsreaktion ist 6,0 ± 0,5, der optimale pH-Wert für die Oxidationsreaktion ist 8,5;
   4) pH-Stabilität:
   zeigt nach einwöchiger Lagerung bei 4 °C und einem pH zwischen 5 und 7,5 eine Restaktivität von 85 %;
   5) Optimale Temperatur:

die optimale Temperatur beträgt 55 °C bei einem pH-Wert von 6,0;
6) Temperaturbeständigkeit:
l5 Minuten bei einem pH-Wert von 6,0 bei 50 °C behandelt beträgt die Restaktivität 90 %;
7) Aktivität:
zeigt eine spezifische Aktivität von ca. 2l00 U/mg Protein;
8) Einfluß von Inhibitoren:
wird durch $HgCl_2$, $CuSO_4$ oder Mercuri-p-chlorbenzoat stark inhibiert;
9) Molekulargewicht:
das Molekulargewicht beträgt 60 000 ± 5 000 (ermittelt durch Gelfiltration);
l0) Molekulargewicht der Untereinheit:
das Molekulargewicht der Untereinheit beträgt 30 000 ± 3 000 (bestimmt durch SDS-Elektrophorese);
ll) $K_M$-Wert;
der $K_M$-Wert für die Reduktionsreaktion für das Substrat Benzoylformiat bei pH 7,0 beträgt 0,22 mM, der $K_M$-Wert für die Oxidationsreaktion für das Substrat D(-)-Mandelat bei pH 8,0 0,5 mM.

**2.** Verfahren zur Gewinnung der D(-)-Mandelat-Dehydrogenase gemäß Anspruch l, dadurch gekennzeichnet, daß man Lactobacillus curvatus DSM 20 0l9 in einem wäßrigen Nährmedium, das eine Quelle für Kohlenstoff und Stickstoff, Thiamin und Mineralsalze enthält, bei einem pH zwischen 5,5 und 6,5 und einer Temperatur zwischen 30 und 37 °C anaerob kultiviert, die Zellmasse abtrennt und das Enzym aus den Zellen isoliert.

**3.** Verwendung der D(-)-Mandelat-Dehydrogenase gemäß Anspruch l zur Herstellung von D(-)-Mandelsäure aus Benzoylformiat.

## Claims

**1.** Microbiologically produced D(-)-mandelate dehydrogenase, characterized by the following physicochemical properties:
1) Reactivity:
it reacts with benzoyl formate in the presence of NADH (nicotinamide adenine dinucleotide) to form D(-)-mandelate and with D(-)-mandelate in the presence of $NAD^+$ to form benzoyl formate;
2) Substrate specificity:
it reduces benzoyl formate particularly well along with other aliphatic and araliphatic 2-ketocarboxylic acids and oxidizes D(-)-mandelate particularly well along with other aliphatic and araliphatic D-2-hydroxycarboxylic acids;
3) Optimal pH value:
the optimal pH value for the reduction reaction is 6.0 ± 0.5 and the optimal pH value for the oxidation reaction is 8.5;
4) pH stability:
shows a residual activity of 85% after storage for 1 week at 4°C and at a pH of 5 to 7.5;
5) Optimal temperature:
the optimal temperature is 55°C at a pH value of 6.0;
6) Temperature stability:
aftertreatment for 15 minutes at a pH value of 6.0 and a temperature of 50°C, the residual activity is 90%;
7) Activity:
shows a specific activity of approx. 2100 U/mg protein;
8) Influence of inhibitors:
is strongly inhibited by $HgCl_2$, $CuSO_4$ or mercury p-chlorobenzoate;
9) Molecular weight:
the molecular weight is 60,000 ± 5,000 (as determined by gel filtration;
10) Molecular weight of the sub-unit: the molecular weight of the sub-unit is 30,000 ± 3,000 (as determined by SDS electrophoresis);
11) $K_M$ value:
the $K_M$ value for the reduction reaction for the substrate benzoyl formate at pH 7.0 is 0.22 mM, the $K_M$ value for the oxidation reaction for the substrate D(-)-mandelate at pH 8.0 is 0.5 mM.

2. A process for the preparation of the D(-)-mandelate dehydrogenase claimed in claim 1, characterized in that Lactobacillus curvatus DSM 20 019 is anaerobically cultivated in an aqueous nutrient medium containing a source for carbon and nitrogen, thiamine and mineral salts at a pH value of 5.5 to 6.5 and at a temperature of 30 to 37°C, the cell mass is separated off and the enzyme is isolated from the cells.

3. The use of the D(-)-mandelate dehydrogenase claimed in claim 1 for the production of D(-)-mandelic acid from benzoyl formate.

**Revendications**

1. Déshydrogénase de D(-)-mandélate préparée microbiologiquement, caractérisée par les propriétés physico-chimiques suivantes :
   1) Réactivité :
   Elle réagit en présence de NADH (nicotinamide-adéninedinucléotide) avec du formiate de benzoyle avec formation de D(-)-mandélate et en présence de $NAD^+$ avec du D(-)-mandélate en formant du formiate de benzoyle,
   2) Spécificité du substrat :
   Elle réduit particulièrement bien du formiate de benzoyle, et en plus de cela d'autres acides 2-céto-carboxyliques aliphatiques ou araliphatiques, et oxyde particulièrement bien du D(-)-mandélate, en plus de cela d'autres acides D-2-hydroxycarbocyliques,
   3) Valeur optimale de pH :
   La valeur optimale de pH pour la réaction de réduction est de 6,0 ± 0,5, la valeur optimale de pH pour la réaction d'oxydation est d 8,5,
   4) Stabilité du pH :
   Elle montre après un stockage d'une semaine à 4°C et un pH entre 5 et 7,5 une activité résiduelle de 85 %,
   5) Température optimale :
   La température optimale se monte à 55°C pour une valeur de pH de 6,0,
   6) Stabilité à la température :
   Traitée 15 minutes à 50°C pour une valeur de pH de 6,0, l'activité résiduelle se monte à 90 %,
   7) Activité :
   Elle monte une activité spécifique d'environ 2100 U/mg de protéine,
   8) Influence des inhibiteurs :
   Elle est inhibée fortement par $HfCl_2$, $CuSO_4$ ou du p-chlorobenzoate de mercure,
   9) Poids moléculaire :
   Le poids moléculaire se monte à 60000 ± 5000 (déterminé par filtration de gel),
   10) Poids moléculaire de l'unité secondair :
   Le poids moléculaire de l'unité secondaire se monte à 30000 ± 3000 (déterminé par électrophorèse SDS),
   11) Valeur de $K_M$ :
   La valeur de $K_M$ pour la réaction de réduction du substrat de formiate de benzoyle à pH 7,0 se monte à 0,22 mmol, la valeur de $K_M$-mandélate à pH 8,0 se monte à 0,5 mmol.

2. Procédé pour l'obtention de la déshydrogénase de D(-)-mandélate selon la revendication 1, caractérisé en ce qu'on cultive de façon anaérobie du lactobacillus curvatus DSM 20019 à un pH compris entre 5,5 et 6,5 et une température entre 30 et 37°C dans un milieu de culture aqueux, qui contient une source pour carbone et azote, thiamine et sels minéraux, en ce qu'on sépare la masse cellulaire et en ce qu'on isole l'enzyme des cellules.

3. Utilisation de la déshydrogénase du D(-)-mandélate selon la revendication 1 pour préparer du D(-)-mandélate à partir du formiate de benzoyle.